Europäisches Patentamt

(19) European Patent Office

Office européen des brevets

(11) Veröffentlichungsnummer: **0 028 325**
**B1**

(12) # EUROPÄISCHE PATENTSCHRIFT

(45) Veröffentlichungstag der Patentschrift:
19.06.85

(51) Int. Cl.⁴: **A 61 B 10/00,** G 01 N 29/00

(21) Anmeldenummer: **80106185.4**

(22) Anmeldetag: **10.10.80**

(54) **Ultraschallgerät für Sektorabtastung.**

(30) Priorität: **16.10.79 DE 2941876**

(43) Veröffentlichungstag der Anmeldung:
**13.05.81 Patentblatt 81/19**

(45) Bekanntmachung des Hinweises auf die Patenterteilung:
**19.06.85 Patentblatt 85/25**

(84) Benannte Vertragsstaaten:
**AT FR GB NL**

(56) Entgegenhaltungen:
**DE - A - 2 448 595**
**DE - B - 2 601 559**
**DE - C - 2 529 155**
**GB - A - 2 015 735**
**US - A - 4 034 744**
**US - A - 4 106 492**

(73) Patentinhaber: **Siemens Aktiengesellschaft, Berlin und München Wittelsbacherplatz 2, D-8000 München 2 (DE)**

(72) Erfinder: **Hagen, Jürgen, Lindenweg 11, D-8521 Grossenseebach (DE)**
Erfinder: **Hetz, Walter, Adam-Kraft-Strasse 17, D-8520 Erlangen (DE)**
Erfinder: **Hetzel, Gert, Dipl.-Ing., Grundherrstrasse 24, D-8520 Erlangen (DE)**
Erfinder: **Schröder, Dirk, Badstrasse 12, D-8520 Erlangen (DE)**

## Beschreibung

Die Erfindung bezieht sich auf ein Ultraschallgerät für Sektorabtastung mit einem Ultraschallkopf, der durch Motorantrieb innerhalb eines vorgegebenen Schwenkwinkelbereiches hin- und herschwenkbar ist, wobei der Motorantrieb einen Antriebsmotor mit Übersetzungsgetriebe und einen Winkelgeber umfaßt, der als Istwertgeber zusammen mit einem Sollwertgeber in einem Regelkreis für den Antriebsmotor zur Erzielung einer Winkelbewegung des Ultraschallkopfes nach Maßgabe des Sollwertes eingeschaltet ist, vgl. US-A-4 106 492.

Der Applikator eines mechanischen Sektorabtasters, insbesondere eines solchen für Ultraschallschnittbilduntersuchungen des menschlichen Herzens, soll im Idealfall so beschaffen sein, daß der Ultraschallkopf mit vorgebbarer, insbesondere konstanter, Winkelgeschwindigkeit bewegt werden kann. Außerdem sollen Bewegungsdurchlauf, Bildfrequenz und maximaler Winkel leicht durch elektronische Ansteuerung geändert werden können. Auch soll der Ultraschallkopf insbesondere für herzphasengetriggerte Einzelbildfotografie aus einer Ruhelage heraus in kürzester Zeit zur gewünschten, insbesondere konstanten, Winkelgeschwindigkeit hochgefahren werden können. Darüber hinaus soll der Antrieb auch immer außerhalb der Flüssigkeit liegen, die z. B. als Vorlaufstrecke verwendet wird, und er soll eine hohe Lebensdauer besitzen. Weiterhin soll ein Ultraschallkopf-Wechsel leicht und schnell durchgeführt werden können. Dabei soll der Applikator jedoch insgesamt klein und kompakt sowie leicht und möglicherweise abgewinkelt sein, so daß er in jeder Körperlage gut applizierbar ist.

Die meisten bekannten Antriebssysteme (z. B. DE-A-2 750 160, DE-A-2 216 577, DE-B-2 601 559, DE-C-2 529 155, US-A-4 034 744, »Ultrasonics«, Juli 1978, S. 171 bis 178) erfüllen die genannten Bedingungen nur ungenügend. So können Antriebssysteme, die beispielsweise mit Schubkurbel- oder Rotationsantrieb oder mit Spiegelablenkung arbeiten, nicht schnell genug zur gewünschten, insbesondere konstanten, Winkelgeschwindigkeit hochgefahren werden. Bei elektromagnetischen Systemen nach dem Prinzip des Drehspulinstruments ist ein rasches Hochfahren im Prinzip möglich; jedoch bereiten Antrieb und Winkelerfassung für größere Sektorwinkel und höhere Bildfrequenz nicht unerhebliche Schwierigkeiten. Nachteilig ist häufig auch, daß der Ultraschallkopf Bestandteil des eigentlichen Antriebssystems ist und somit das Antriebssystem und der Winkelgeber in der Flüssigkeit der Vorlaufstrecke liegen müssen, so daß ein schneller Ultraschallkopf-Wechsel nicht möglich ist.

Vorliegende Erfindung geht aus von einem Ultraschallgerät der eingangs genannten Art, wie es beispielsweise durch die US-A-4 106 492 vorbekannt ist. Nach Spalte 3, Zeilen 21 und 22 der genannten US-A-4 106 492 erlaubt dieses Ultraschallgerät jedoch lediglich programmierte Schwenkbewegungen des Ultraschallkopfes in Winkelbereichen zwischen +25° und −25°. Ein solcher Schwenkwinkelbereich ist jedoch für eine vielseitige Anwendung des Abtastgerätes zu eng. Erstrebenswert sind Schwenkwinkelbereiche von wenigstens +40° bis −40° und darüber.

Aus der GB-A-2 015 735 ist ein Ultraschallgerät zur Abtastung eines Körpers bekannt, das — im Gegensatz zum vorliegenden Ultraschallgerät — mit zwei oder mehr Ultraschall-Übertragern arbeitet, die an einem gemeinsamen zylindrischen Ultraschallkopf befestigt sind sowie im Betrieb jeweils kontinuierlich und mit fester Winkelgeschwindigkeit um 360° um die Ultraschallkopfachse gedreht werden. Ein Hin- und Herschwenken oder Pendeln des Ultraschallkopfes findet hier also nicht statt. Die Winkelstellung des zylindrischen Ultraschallkopfes in Form eines $\cos \alpha$- und eines $\sin \alpha$-Signals wird für Zwecke der Bilddarstellung mit Hilfe eines Stellungsgebers ermittelt. Da die einzelnen Ultraschall-Übertrager am Ultraschallkopf normalerweise nicht hinreichend genau positioniert werden können, ist eine elektrische Korrekturschaltung vorgesehen, die solche Fertigungstoleranzen korrigiert. Nach Figur 7 dieser Druckschrift enthält die Korrekturschaltung u. a. einen Oszillator, der eine Dreiecksspannung abgibt. Mit Hilfe von zwei Verstärkern wird die Dreieckspannung in zwei Signale mit rechteckförmigem Zeitverlauf umgesetzt, die einer Logikschaltung zugeführt werden. Die Logikschaltung wirkt auf die Bilddarstellung ein; sie ist also nicht für Zwecke der Regelung des Antriebsmotors vorgesehen.

Aufgabe der vorliegenden Erfindung ist es, ein Ultraschallgerät für Sektorabtastung der eingangs genannten Art anzugeben, das in einem erweiterten Schwenkwinkelbereich des Ultraschallkopfes einwandfrei arbeitet.

Die Aufgabe wird erfindungsgemäß dadurch gelöst, daß der Sollwertgeber in Abhängigkeit von der Zeit eine Linearfunktion liefert, und daß der Winkelgeber ein Linearwinkelgeber ist, der zumindest innerhalb des Schwenkwinkelbereiches ebenfalls eine Linearfunktion liefert.

Der Erfindung gingen Überlegungen voraus, warum wohl das Ultraschallgerät der US-A-4 106 492 im Konzept lediglich Schwenkwinkel im Bereich von −25° bis +25° zuläßt. Es wurde gefunden, daß beim Gegenstand der US-A-4 106 492 im Istwert-Sollwert-Vergleich zwei Funktionen miteinander verglichen werden, die in der Form am Regeleingang nicht unerheblich voneinander abweichen. Die beim Gegenstand der US-A-4 106 492 als Istwert auftretende Sinusfunktion und die als Dreieck vorgegebene Sollwertfunktion sind lediglich für kleine Winkel, d. h. in einem sehr kleinen Signalbereich, formgleich. Durch die vorliegende Erfindung ergibt sich jedoch innerhalb des gewünschten Schwenkwinkelbereiches bereits am Regeleingang Formgleichheit über einen weiten Signalbereich. Damit folgt der Istwert dem linearen Sollwert exakt über einen gegenüber vorher wesentlich breiteren Winkelbereich, beispielsweise in dem vorliegend angestrebten Schwenkwinkelbereich von wenigstens +40° bis −40° und darüber, so daß das Gerät gemäß der Erfindung für einen viel breiteren Winkelbereich als bisher anwendbar ist. Eine gleichförmige Winkelgeschwindigkeit im gesamten Schwenkwinkelbereich wird erzielt, weil der Sollwertgeber eine Linearfunktion liefert und weil als Winkelgeber ein Linearwinkelgeber eingesetzt wird. Dessen Winkelerfassungsbereich sollte in der Linearstrecke zumindest über jene eines üblichen Sinus- oder Kosinusgebers hinausreichen, vorzugsweise jedoch im gesamten Bereich linear verlaufen.

Der Linearwinkelgeber kann dabei aus einem Sinus- oder Kosinusgeber mit nachgeschaltetem Linearglied bestehen; er kann aber auch direkt ein Winkelerfassungsglied sein, das den Winkel direkt in linearer Funktion erfaßt. Als Winkelgeber empfehlen sich in diesem Falle Feldplattenpotentiometer oder auch Resolver.

In weiterer vorteilhafter Ausgestaltung der Erfindung sollen Antriebsmotor, Winkelgeber und Ultraschallkopf auch über Antriebsriemen miteinander verbunden sein, die am Antriebsmotor, am Winkelgeber und am Ultraschallkopf vorgesehenen Antriebsräder wenigstens teilweise umschlingen. Der Antriebsriemen sollte zweckmäßigerweise ein Zahnriemen sein, der in teilweiser Umschlingung an den Antriebszahnrädern mit einer Vielzahl von Zähnen eingreift.

Schließlich kann in vorteilhafter Ausgestaltung der Erfindung der Ultraschallkopf auch als Mehrfachschallkopf ausgebildet sein, wobei am Umfang des Ultraschallkopfes versetzt angeordnete Ultraschallschwinger über den Motorantrieb nach Durchführung einer entsprechenden Drehbewegung in die gewünschte Abtastposition einfahrbar sind. Damit ergibt sich die Möglichkeit, durch einfaches Drehen unterschiedliche Ultraschallschwinger mit z. B. unterschiedlichen Frequenzen zur Erzielung unterschiedlicher Eindringtiefen im Untersuchungsobjekt zur Sektorabtastung ohne Auswechslung des kompletten Ultraschallkopfes einzusetzen. Wird als Winkelgeber in einem solchen Falle ein Feldplattenpotentiometer eingesetzt, so ergibt sich insbesondere im Falle eines Doppelschallkopfes, bei dem eine 180°-Drehbewegung zum Einfahren eines neuen Schwingers in eine Abtastposition benötigt wird, eine lückenlose Regelung, da ein Feldplattenpotentiometer lediglich aufgrund einfacher Umpolung 180°-Periodizität der Ausgangsfunktion aufweist.

Weitere Vorteile und Einzelheiten der Erfindung ergeben sich aus der nachfolgenden Beschreibung von Ausführungsbeispielen anhand der Zeichnung in Verbindung mit den Unteransprüchen.

Es zeigt

Fig. 1 einen Ultraschallapplikator für ein Ultraschallgerät nach der Erfindung im Prinzipaufbau,

Fig. 2 ein Prinzipschaltbild des Ultraschallgerätes mit einem Applikator gemäß der Figur 1,

Fig. 3 eine erste Modifikation des Schaltbildes der Figur 2,

Fig. 4 eine zweite Modifikation des Schaltbildes der Figur 2,

Fig. 5 bis 7 Modifikationen des Ausführungsbeispieles der Figur 1,

Fig. 8 das Beispiel eines Dreifachschallkopfes zum modifizierten Einsatz in einem der Ausführungsbeispiele der Figuren 1 bis 7.

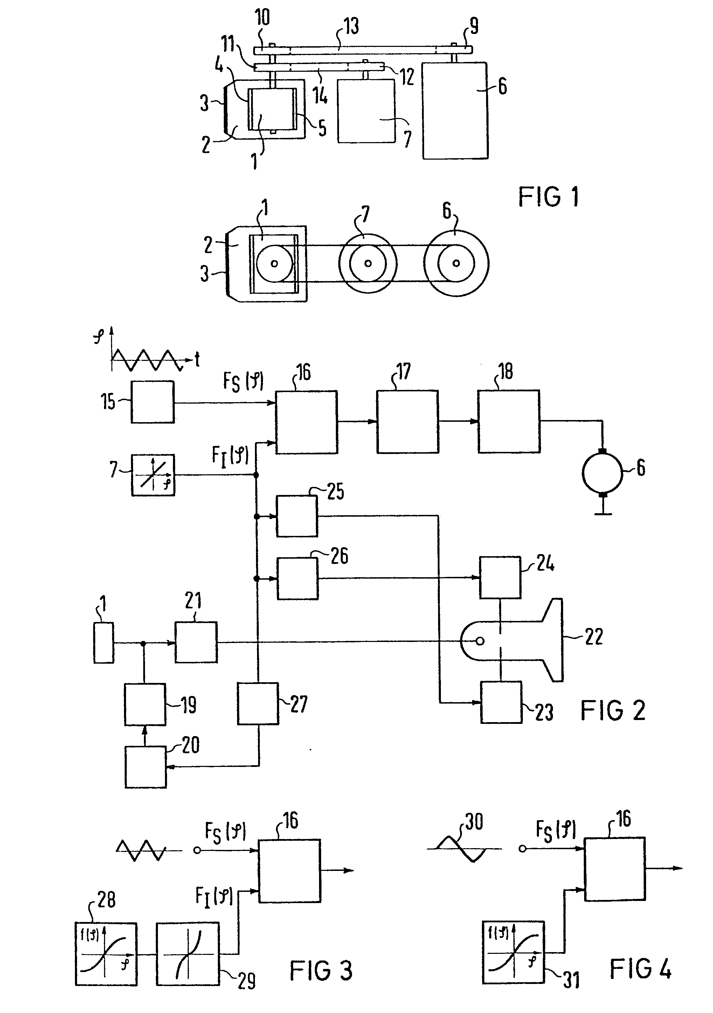

In der Figur 1 ist ein Ultraschallkopf mit 1 bezeichnet. Er befindet sich in einem Behälter 2, der mit einer Ankoppelflüssigkeit, z. B. Wasser oder Öl, gefüllt ist. Als Austrittsfenster für die Ultraschallwellen dient eine stirnseitige Membran 3 des Flüssigkeitsbehälters 2. Der Ultraschallkopf 1 beinhaltet im vorliegenden Ausführungsbeispiel insgesamt zwei hinsichtlich seiner Drehachse diametral gegenüberliegende Schwingerelemente 4 und 5. Zum Antrieb des Ultraschallkopfes 1 im Sinne der Erzeugung einer Winkelschwenkbewegung ist ein Antriebsmotor 6 vorgesehen; der Istwert des Schwenkwinkels wird über einen Winkelgeber 7 erfaßt. Die betriebsmäßige Verbindung zwischen dem Ultraschallkopf 1 und dem Antriebsmotor 6 sowie dem Winkelgeber 7 erfolgt über ein aus Zahnrädern 9 bis 12 sowie Zahnriemen 13 und 14 bestehendes Übersetzungsgetriebe.

Eine Betriebsschaltung für den Ultraschall-Applikator nach Figur 1 ist in Figur 2 dargestellt. In Figur 2 sind der Ultraschallkopf 1 und der Antriebsmotor 6 schematisch eingezeichnet. Dem Winkelgeber 7 für den Istwert ist ein Sollwertgeber 15 zugeordnet, dessen Sollwertsignal $F_S(\varphi)$ zusammen mit dem Istwertsignal $F_I(\varphi)$ des Winkelgebers 7 einem Differenzbildner 16 zugeleitet wird. Über einen PID-Regler 17 und dann über eine Leistungsstufe 18 wird der Lauf des Antriebsmotors 6 auf den Sollwert eingeregelt.

Zur Erregung des Ultraschallkopfes 1 zwecks Abstrahlung von Ultraschallsendeimpulsen während der Sektorabtastung dient in der üblichen Weise ein Hochfrequenzimpulsgenerator 19, der im Takt eines Leittaktgebers oder Leitgenerators 20 dem Ultraschallkopf 1 Erregungsimpulse zuleitet. Die nach Aussenden entlang einer Zeile im Sektorfeld anfallenden Ultraschallechosignale werden vom Ultraschallkopf 1 rückgewandelt, und die rückgewandelten elektrischen Signale werden nach Verstärkung in einem Empfangsverstärker 21 schließlich auf die Hellmoduliereinrichtung einer Bildröhre 22 zum Aufbau eines Echosichtbildes gegeben. Die zum Bildaufbau notwendige Zeilenkippspannung wird von einem Zeilenkippgenerator 23 und die erforderliche Bildkippspannung von einem Bildkippgenerator 24 geliefert. Grundelemente zur Erzeugung der Kippspannungen sind dabei ein Sinusgeber 25 und ein Kosinusgeber 26, denen eingangsseitig das Istwertsignal $F_I(\varphi)$ des Istwertwinkelgebers 7 zugeleitet wird.

Die Synchronisation des Leittaktes des Leittaktgebers 20 erfolgt ebenfalls in Abhängigkeit des Istwertsignales $F_I(\varphi)$ über einen Komparator 27 (oder über einen Analog-Digital-Wandler), der ausgehend von einer bestimmten Amplitude des Istwertsignales bei vorgebbaren Änderungsschritten bis zu einer zweiten Amplitude des Istwertsignals den Taktgeber 20 im Sinne der Erzeugung von Leittaktimpulsen steuert. Zur Verbesserung der Bildqualität können bei Bedarf hiermit auch die Umkehrpunkte der Sektorschwenkbewegung ausgespart werden. Dabei ergibt sich auch eine ortsfeste Lage der Bildzeilen auf dem Bildschirm der Bildröhre 22.

Im Ausführungsbeispiel nach Figur 2 ist die Sollwertfunktion $F_S(\varphi)$ des Sollwertgebers 15 eine Linearfunktion in Sägezahnform. Beim Winkelgeber 7 handelt es sich gemäß der Erfindung um einen Linearwinkelgeber, dessen Linearstrecke im Winkelerfassungsbereich zumindest über jene eines üblichen Sinus- oder Kosinusgebers hinausreicht oder im gesamten Bereich linear verläuft. Als Winkelgeber 7 dient im vorliegenden Fall ein Feldplattenpotentiometer; es können selbstverständlich auch lineare kapazitive oder induktive Winkelgeber oder auch lineare Resolver eingesetzt werden. Die beschriebene Ausführungsform gewährleistet Linearität über einen weiten Winkelbereich, z. B. zwischen +40° und —40° oder darüber hinaus. Die Vorgabe der Linearfunktion $F_S(\varphi)$ hat den Vorteil, daß über den gesamten Schwenkwinkelbereich die Winkelgeschwindigkeit der Schwenkbewegung des Ultraschallkopfes 1 konstant ist. Daraus resultiert gleichbleibende Informationsdichte.

Figur 3 zeigt in einer Modifikation des Prinzipschaltbildes der Figur 2 wieder einen sägezahnförmigen Verlauf der Sollwertspannung $F_S(\varphi)$ am Eingang des Differenzbildners 16. Der Winkelgeber 7 weist jedoch nicht von vornherein eine lineare Kennlinie auf; vielmehr dient als Winkelgeber 7 hier ein Sinus-(oder Kosinus-)Winkelgeber 28 in Verbindung mit einem nachgeschalteten Linearisierglied 29. Es tritt damit derselbe Linearisierungseffekt auf wie beim Linear-Winkelgeber 7 der Fig. 2.

Die Figur 4 zeigt eine Modifikation in dem Sinne, daß als Sollwertsignal $F_S(\varphi)$ eine Sinus- oder Kosinusfunktion vorgegeben wird, also eine solche Funktion, wie sie mit etwas angespitzten Amplituden mit der Kennziffer 30 dargestellt ist. Als Istwertgeber kann dann unmittelbar ein Sinus-(bzw. Kosinus-)Winkelgeber 31 dienen.

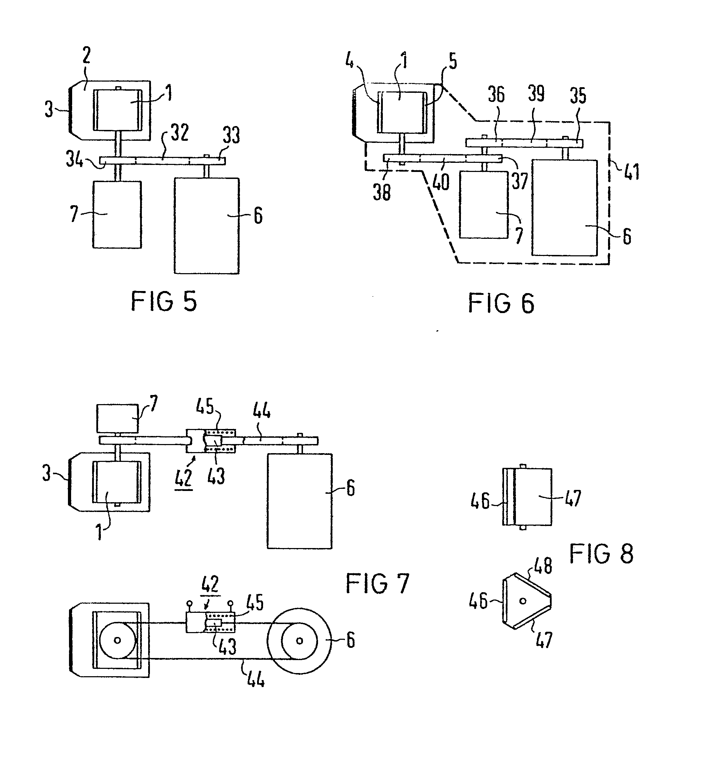

Entsprechend dem Schaltbild der Figur 2 kann selbstverständlich auch die räumliche Anordnung von Ultraschallkopf 1, Antriebsmotor 6 und Winkelgeber 7 zueinander in zweckmäßiger Weise verändert werden. Ein erstes Modifikationsbeispiel zeigt Figur 5, bei dem der Antriebsmotor 6 über einen einzelnen gemeinsamen Zahnriemen 32 mit Zahnrädern 33 und 34 den Ultraschallkopf 1 und den Winkelgeber 7 um die gemeinsame Drehachse dreht.

Das Beispiel nach Figur 6 zeigt eine Anordnung von Ultraschallkopf 1, Antriebsmotor 6, Winkelgeber 7 und Zahnriemengetriebe 35 bis 40 in der Weise, daß sich bei Ummantelung mit dem Gehäuse 41 die winklig abgeknickte Form zwischen dem vorderen Schallkopfgehäuse und dem Handstück mit Antriebsmotor 6 und Winkelgeber 7 ergibt. Das Ausführungsbeispiel nach Figur 6 stellt also eine für die Sektorabtastung besonders handliche Ausbildungsform dar, die neben der üblichen präkardialen Anwendung unabhängig auch für suprasternalen und subxiphoidalen Einsatz geeignet ist.

In sämtlichen der bisher beschriebenen Beispiele kommt als Antriebsmotor 6 vorzugsweise ein sogenannter Glockenankermotor in Frage. Ein solcher Motor erfüllt besonders gut die an ihn hinsichtlich Leichtigkeit des Laufes, rasche und sichere Umschaltbarkeit der Drehrichtung, gute Steuer- bzw. Regelbarkeit gerichteten Bedingungen. Glockenankermotoren, die für den Einsatz geeignet sind, sind im Handel erhältlich (z. B. Prospekt der Fa. Faulhaber, DC-Micromotoren Serien 2225 bis 3557).

Anstelle von Antriebsmotoren, wie beispielsweise einem Glockenankermotor, können selbstverständlich auch andere rasch umschaltbare Antriebe, wie insbesondere elektromagnetische Antriebe, eingesetzt werden. Ein Ausführungsbeispiel, das mit einem elektromagnetischen Antrieb 42 arbeitet, ist in Figur 7 dargestellt. Der elektromagnetische Antrieb 42 besteht dabei aus einem Dauermagneten 43, der fest mit einem Zahnriemen 44 verbunden ist. Die Regelung steuert Polarität und Stromfluß durch eine Erregerspule 45. Durch das infolge des Stromflusses entstehende magnetische Feld wird eine Kraft auf den Dauermagneten 43 ausgeübt. Die Kraftrichtung ist von der Stromrichtung abhängig. Der Winkelgeber 7 ist im vorliegenden Fall wieder ein Drehwinkelgeber. Er kann jedoch ohne weiteres durch einen geeigneten linearen Weggeber ersetzt werden.

Sämtliche der beschriebenen Ausführungsbeispiele haben den Vorteil, daß der Ultraschallkopf 1 nicht mehr Teil des Antriebssystems ist; das Antriebselement 6 und das Glied 7 zur Winkelerfassung können also immer außerhalb der Flüssigkeit des Flüssigkeitsbehälters angeordnet sein. Antreibendes Element ist ein elektronisch regelbarer Antrieb, der den Ultraschallkopf 1 so bewegt, daß er in Pendelbewegung im vorgegebenen Winkelbereich einer vorgegebenen Zeitfunktion folgt. Durch die hier speziell gewählte Regelung ergibt sich ein Bewegungsverlauf der Pendelbewegung (Istwert) mit konstanter Winkelgeschwindigkeit. Bildfrequenz und maximaler Sektorwinkel lassen sich vorgeben. Aufgrund der gewählten Konzeption können die zu bewegenden Massen sehr klein gehalten werden. Hierdurch wird gewährleistet, daß der Ultraschallkopf über die Antriebselemente rasch in der Drehrichtung umgesteuert werden kann, und daß insbesondere für herzphasengetriggerte Fotografie der Ultraschallkopf 1 sehr rasch aus der Ruhestellung zu einem Bildsuchlauf gesteuert werden kann.

In sämtlchen Ausführungsbeispielen umfaßt der Ultraschallkopf 1 jeweils zwei Ultraschallschwinger oder Schwingerelemente 4 und 5, die hinsichtlich der Schwenkachse diametral gegenüberliegend angeordnet sind. Die beiden Schwingerelemente 4, 5 können sich hinsichtlich Frequenz, Fokuslage und Durchmesser unterscheiden. Bei einer 180°-Drehung kann somit durch Anbringen eines geeigneten Schwingerelements vor der Austrittsmembran 3 des Ultraschallapplikators auf eine neue Betriebsart umgeschaltet werden. Es können damit, lediglich durch einfaches und rasches Umschalten, verschiedene Tiefen des Untersuchungsobjektes nach unterschiedlichen Ultraschallbedingungen ausgelotet werden. Ein Auswechseln des kompletten Applikators ist also nicht erforderlich.

Die Anordnung zweier Schwingerelemente 4 und 5 ist selbstverständlich nicht immer notwendig; sofern der Untersuchungsvorgang mit einem einzigen Schwingerelement auskommt, kann der Ultraschallkopf 1 natürlich auch nur mit einem einzigen Schwingerelement versehen sein. Ebensogut ist bei Bedarf auch eine Erweiterung der Schwingerzahl über zwei hinaus möglich. Figur 8 zeigt beispielsweise schematisch einen Ultraschallkopf mit insgesamt drei um jeweils 120° gegeneinander versetzten Ultraschall-Schwingerelementen 46, 47 und 48.

Um bei den Ausführungsbeispielen mit dem Antriebsmotor 6 eine höhere Lebensdauer des Motors 6 zu gewährleisten, ist die Nutzung eines Drehbereiches, der größer ist als der Schwenkbereich des Ultraschallkopfes, vorteilhaft. Hierzu braucht lediglich eine geeignete mechanische Untersetzung 1 : n der Schallkopfbewegung zur Motordrehung gewählt werden. Beispielsweise kann n = 2 gewählt werden, so daß also eine Drehung des Ultraschallkopfes um 90° einer Motordrehung um 180° entspricht.

Die beschriebenen Ausführungsformen können im Rahmen der Erfindung modifiziert werden. So kann insgesamt die Anordnung einzelner Bauteile im Hinblick auf Handlichkeit und Kompaktheit nach Bedarf verändert werden. Hierzu gehören auch Ausführungsformen, bei denen Motor, Winkelgeber und Ultraschallkopf auf einer Drehachse sitzen. Bei einer solchen Ausführungsform, die z. B. für suprasternale Applikation denkbar ist, kann bei Bedarf auf eine zusätzliche Übersetzung zwischen den Einzelelementen verzichtet werden.

**Patentansprüche**

1. Ultraschallgerät für Sektorabtastung mit einem Ultraschallkopf (1), der durch den Motoran-trieb innerhalb eines vorgegebenen Schwenk-winkelbereiches hin- und herschwenkbar ist, wobei der Motorantrieb einen Antriebsmotor (6) mit Übersetzungsgetriebe und einen Winkelgeber (7) umfaßt, der als Istwertgeber zusammen mit einem Sollwertgeber (15) in einem Regelkreis für den Antriebsmotor (6) zur Erzielung einer Winkelbewegung des Ultraschallkopfes nach Maßgabe des Sollwertgebers eingeschaltet ist, dadurch gekennzeichnet, daß der Sollwertgeber (15) in Abhängigkeit von der Zeit (t) eine Linearfunktion liefert, und daß der Winkelgeber (7; 28, 29) ein Linearwinkelgeber ist, der zumindest innerhalb des Schwenkwinkelbereiches ebenfalls eine Linearfunktion liefert.

2. Ultraschallgerät nach Anspruch 1, dadurch gekennzeichnet, daß der Linearwinkelgeber (7) aus einem Sinus- oder Kosinusgeber (28) mit ei-nem nachgeschalteten Linearisierglied (29) be-steht, oder daß der Linearwinkelgeber (7) ein Winkelerfassungsglied ist, das direkt den Schwenkwinkel in linearer Funktion erfaßt.

3. Ultraschallgerät nach Anspruch 2, dadurch gekennzeichnet, daß der Winkelgeber (7) ein Feldplattenpotentiometer oder ein linearer Resolver ist.

4. Ultraschallgerät nach einem der Ansprüche 1 bis 3, dadurch gekennzeichnet, daß über ein Bauglied (27) der Sendetakt für Ultraschallsendeimpulse vom Istwertsignal ($F_I(\varphi)$) gesteuert ist in der Weise, daß ausgehend von einer bestimmten Amplitude des Istwertsignals bei vorgebbaren Änderungsschritten bis zu einer zweiten Amplitude des Istwertsignals ein Taktgeber (20) im Sinne der Erzeugung von Sendeleittaktimpulsen gesteuert wird.

5. Ultraschallgerät nach einem der Ansprüche 1 bis 4, dadurch gekennzeichnet, daß Bestandteil des Übersetzungsgetriebes des regelbaren Motorantriebes ein System aus Antriebsriemen (13, 14; 32; 39, 40; 44) ist, das an der Riemenauflage Antriebsglieder (9 bis 12; 33, 34; 35 bis 38) auf Seiten des Antriebsmotors (6) einerseits sowie Antriebsglieder auf Seiten des Ultraschallkopfes (1) oder des Winkelgebers (7) andererseits wenigstens teilweise umschlingt.

6. Ultraschallgerät nach Anspruch 5, dadurch gekennzeichnet, daß als Antriebsriemen (13, 14; 32; 39, 40; 44) Zahnriemen dienen, die in teilweiser Umschlingung von Antriebszahnrädern oder Radsegmenten auf Seiten des Antriebsmotors (6) und auf Seiten des Ultraschallkopfes (1) oder Winkelgebers (7) in deren Zahnkränze mit einer Vielzahl von Zähnen eingreifen.

7. Ultraschallgerät nach einem der Ansprüche 1 bis 6, dadurch gekennzeichnet, daß der Ultraschallkopf (1) als Mehrfachschallkopf mit einer Mehrzahl von Schwingern (4, 5; 46, 47, 48) ausgebildet ist, die durch den Motorantrieb durch Drehung um die Schwenkachse in den Applikationsbereich einschwenkbar ausgebildet sind, so daß zu einem beliebigen Zeitpunkt ein beliebiger Schwinger (4, 5) als Aktivschwinger des Ultraschallkopfes (1) einschaltbar ist.

8. Ultraschallgerät nach Anspruch 7, dadurch gekennzeichnet, daß mindestens zwei Schwinger (4, 5; 45, 46, 48) vorhanden sind, die zum Betrieb mit unterschiedlicher Ultraschallfrequenz vorgesehen sind.

9. Ultraschallgerät nach einem der Ansprüche 5 oder 6, dadurch gekennzeichnet, daß ein erster Antriebsriemen (39) zwischen Winkelgeber (7) und dem Antriebsmotor (6) sowie ein zweiter Antriebsriemen (40) zwischen dem Winkelgeber (7) und dem Ultraschallkopf (1) vorgesehen ist (Fig. 6).

10. Ultraschallgerät nach einem der Ansprüche 1 bis 9, dadurch gekennzeichnet, daß der Sollwertgeber (15) zur Abgabe einer Linearfunktion in Sägezahnform vorgesehen ist.

## Claims

1. A ultrasonic sector scanning device, with an ultrasonic head (1) which can be pivoted back and forth by a motor drive within a given pivot angle range, where the motor drive comprises a drive motor (6) having a step-up gear and an angle transmitter (7), which is connected into a regulating circuit for the drive motor (6) as an actual value indicator together with a theoretical value indicator (15) in order to achieve an angular movement of the ultrasonic head in accordance with the theoretical value indicator, characterised in that the theoretical value indicator (15) supplies a linear function in dependence upon the time (t) and that the angle transmitter (7; 28, 29) is a linear angle transmitter which likewise supplies a linear function, at least within the pivot angle range.

2. An ultrasonic device as claimed in Claim 1, characterised in that the linear angle transmitter (7) consists of a sine or cosine transmitter (28) whose output is connected to a linearisation element (29), or that the linear angle transmitter (7) is an angle detector which directly detects the pivot angle in a linear function.

3. An ultrasonic device as claimed in Claim 2, characterised in that the angle transmitter (7) is a field plate potentiometer or a linear resolver.

4. An ultrasonic device as claimed in one of the Claims 1 to 3, characterised in that the transmitting clock pulse train for ultrasonic transmitted pulses is controlled by the actual value signal $(F_I(\varphi))$ via a member (27) in such manner that, commencing from a specific amplitude of the actual value signal, a clock pulse generator (20) is controlled in accordance with the production of transmitted clock pulses at predetermined stages of change up to a second amplitude of the actual value signal.

5. An ultrasonic device as claimed in one of the Claims 1 to 4, characterised in that part of the step-up gear of the regulatable motor drive consists of a system of drive belts (13, 14; 32; 39, 40; 44) on the belt bearing which at least partially embraces drive elements (9 to 12; 33, 34; 35 to 38) on the side of the drive motor (6) on the one hand and drive elements on the side of the ultrasonic head (1) or the angle transmitter (7) on the other hand.

6. An ultrasonic device as claimed in Claim 5, characterised in that the drive belts (13, 14; 32; 39, 40; 44) consist of toothed belts which engage into the toothed rims thereof with a plurality of teeth partially embraced by drive toothed weels or wheel segments on the side of the drive motor (6) and on the side of the ultrasonic head (1) or angle transmitter (7).

7. An ultrasonic device as claimed in one of the Claims 1 to 6, characterised in that the ultrasonic head (1) is designed as a multiple sonic head having a plurality of oscillators (4, 5; 46, 47, 48) which can be pivoted into the application range by the motor drive by rotation about the pivot axis, so that at any time an arbitrary oscillator (4, 5) can be connected as the active oscillator of the ultrasonic head (1).

8. An ultrasonic device as claimed in Claim 7, characterised in that at least two oscillators (4, 5; 45, 46, 48) are provided which serve for operation at different ultrasonic frequencies.

9. An ultrasonic device as claimed in one of the Claims 5 or 6, characterised in that a first drive belt (39) is arranged between the angle transmitter (7) and the drive belt motor (6), and a second drive belt (40) is arranged between the angle transmitter (7) and the ultrasonic head (1) (Fig. 6).

10. An ultrasonic device as claimed in one of the Claims 1 to 9, characterised in that the theoretical value indicator (15) serves to emit a linear function in sawtooth form.

## Revendications

1. Appareil ultrasonique pour l'exploration sectorielle comportant une tête ultrasonique (1), qui peut être basculée selon un mouvement de va-et-vient à l'aide d'un dispositif d'entraînement à moteur à l'interieur d'une plage prédéterminée d'angles de basculement, le dispositif d'entraînement à moteur comportant un moteur d'entraînement (6) muni d'une transmission et d'un capteur angulaire (7), qui est branché en tant que générateur de valeurs instantanées ainsi qu'avec un générateur de valeurs de consigne (15), dans un circuit de réglage du moteur d'entraînement (6) en vue d'obtenir un déplacement angulaire de la tête ultrasonique en fonction du générateur de valeurs de consigne, caractérisé par le fait que le générateur de valeurs de consigne (15) fournit une fonction linéaire et que le capteur angulaire (7; 28, 29) est un capteur angulaire linéaire qui fournit également une fonction linéaire au moins à l'intérieur de la plage des angles de basculement.

2. Appareil ultrasonique selon la revendication 1, caractérisé en ce que le capteur angulaire linéaire (7) est constitué par un générateur en sinus ou en cosinus (28), en aval duquel est branché un circuit de linéarisation (29) ou bien que le capteur angulaire linéaire (7) est un organe de détection d'angles, qui détermine directement l'angle de basculement selon une fonction linéaire.

3. Appareil ultrasonique suivant la revendication 2, caractérisé par le fait que le capteur angulaire (7) est un potentiomètre à magnétorésistance ou un résolveur linéaire.

4. Appareil ultrasonique suivant l'une des revendications 1 à 3, caractérisé par le fait que la cadence d'émission des impulsions d'émission ultrasoniques est commandé par l'intermédiaire d'un composant (27), par le signal de la valeur instantanée ($F_1(\varphi)$) de telle sorte qu'à partir d'une amplitude déterminée du signal de la valeur instantanée et pour des pas de modification pouvant être prédéterminés jusqu'à une seconde amplitude du signal de la valeur instantanée, un générateur de cadence (20) est commandé en vue de produire des impulsions de cadence d'émission.

5. Appareil ultrasonique suivant l'une des revendications 1 à 4, caractérisé par le fait que la transmission du dispositif réglable d'entraînement à moteur comprend un système constitué de courroies d'entraînement (13, 14; 32; 39; 40; 44) qui entourent au moins partiellement d'une part des organes d'entraînement (9 à 12; 33; 34; 35 à 38) du côté du moteur d'entraînement (6) et d'autre part des organes d'entraînement du côté de la tête ultrasonique (1) ou du capteur angulaire (7).

6. Appareil ultrasonique suvant la revendication 5, caractérisé par le fait que l'on utilise comme courroies d'entraînement (13, 14; 32; 39, 40; 44) des courroies dentées qui, en entourant partiellement des roues dentées d'entraînement ou des segments dentés du côté du moteur d'entraînement (6) et du côté de la tête ultrasonique (1) ou du capteur angulaire (7), engrènent dans les couronnes dentées de ces dents, qui comportent un grand nombre de dents.

7. Appareil ultrasonique suivant l'une des revendications 1 à 6, caractérisé par le fait que la tête ultrasonique (1) est réalisée sous la forme d'une tête multiple comportant une multiplicité de transducteurs (4, 5; 46, 47, 48) qui sont réalisés de manière à pouvoir être basculés, au moyen du dispositif d'entraînement à moteur, par rotation autour de l'axe de basculement, dans la zone d'application de telle sorte qu'à un instant quelconque un transducteur quelconque (4, 5) peut être branché en tant transducteur actif de la tête ultrasonique (1).

8. Appareil ultrasonique suivant la revendication 7, caractérisé par le fait qu'il est prévu au moins deux transducteurs (4, 5; 45, 46, 48) qui sont agencés de manière à fonctionner avec des fréquences ultrasoniques différentes.

9. Appareil ultrasonique suivant l'une des revendications 5 ou 6, caractérisé par le fait qu'il est prévu une première courroie d'entraînement (39) entre le capteur angulaire (7) du moteur d'entraînement (6) ainsi qu'une seconde courroie d'entraînement (40) entre le capteur angulaire (7) et la tête ultrasonique (1) (Figure 6).

10. Appareil ultrasonique suivant l'une des revendications 1 à 9, caractérisé par le fait que le générateur des valeurs de consigne (15) est prévu de manière à fournir une fonction linéaire en forme de dents de scie.

0 028 325

FIG 1

FIG 2

FIG 3

FIG 4

FIG 5

FIG 6

FIG 7

FIG 8